# EUROPEAN PATENT SPECIFICATION

(11) **EP 0 635 668 B1**
(45) Date of publication and mention of the grant of the patent: **03.09.1997**
(21) Application number: 94202127.0
(22) Date of filing: 21.07.1994
(51) Int. Cl.: F16L 37/00, F16L 37/40, F16L 37/23

(54) **Connecting device**
Verbindungsvorrichtung
Dispositif de raccordement

(30) Priority: 23.07.1993 NL 9301298
(43) Date of publication of application: 25.01.1995
(73) Proprietor: Portanje Elektronika B.V., Amsterdam (NL)
(72) Inventor: Balk, Annette Hermiene, NL-6401 HL Roermond (NL)
(74) Representative: de Vries, Johannes Hendrik Fokke

(56) References cited:
- DE-A- 1 943 552
- FR-A- 2 074 163
- NL-A- 7 207 850

## Description

The invention relates to a device in accordance with the preamble of appended claim 1.

A device of this kind is known from FR-A-2 074 163. In the known device of this type the connecting element is coupled with the connecting box by inserting the connecting nipple in axial direction into the connecting opening. In this case the connecting element first arrives in a parking position and is locked in this parking position in that the second locking elements engage the collar of the connecting nipple and hold the connecting element. The closing valve remains closed in this parking position so that no gas or vacuum is provided to the user. When the connecting element with the connecting nipple is pressed further into the connecting opening, the connecting element arrives in the working position in which the closing valve is opened so that the user is connected to the gas source or vacuum source. The connecting element is locked in the working position in that the first locking means now engage the collar of the connecting nipple. When one wishes to disengage the user from the gas source or vacuum source, the actuating means is operated to release the locking of the connecting element in the connecting box. Pushing the actuating means forwards from the locked working position releases both the first and the second locking elements, but actuates a separate set of locking elements to prevent the connecting element shooting out. Release of the actuating means then releases this extra set of locking elements but re-locks the second locking elements to hold the connector in the final parking position. The actuating means must be operated a second time to allow removal of the connecting element.

The invention aims to provide a device of the above-mentioned type wherein it is realized in a more simple manner that at operating the actuating means for releasing the locking, the connecting element cannot be forced fully out of the connecting box unintentionally.

To this end the device according to the invention is characterized in accordance with the characterizing portion of claim 1.

In this manner it is obtained that at releasing the locking of the connecting element in the working position, the connecting element will be blocked in the non-working position with certainty.

The invention will be further explained hereinafter by reference to the drawings in which an embodiment of the device according to the invention is schematically shown.

Fig. 1 shows schematically an axial section of an embodiment of the device according to the invention, wherein the connecting element is located in the working position within the connecting box.

Fig. 2 is an axial section corresponding with Fig. 1, wherein the connecting element is located in the parking position within the connecting box.

Fig. 3 is an axial section of the connecting box of the device of Fig. 1.

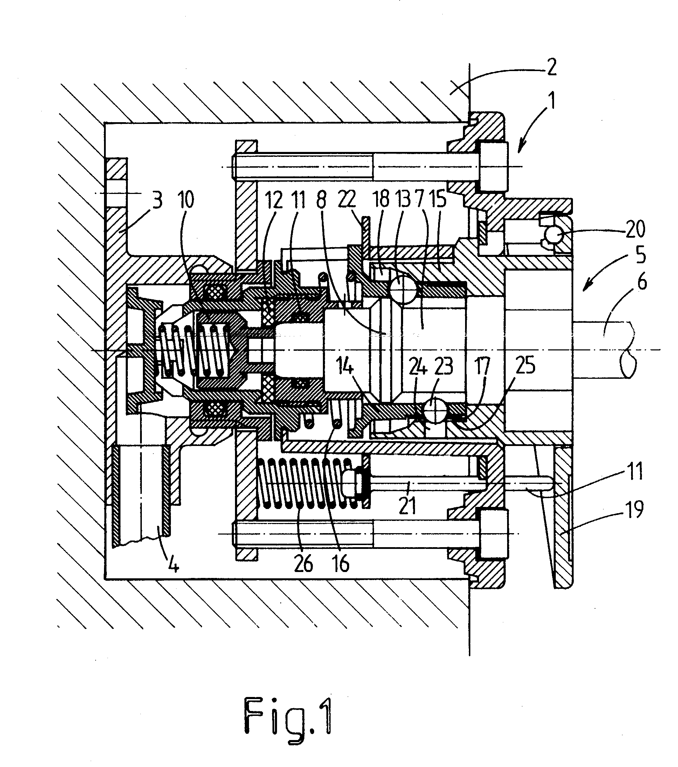

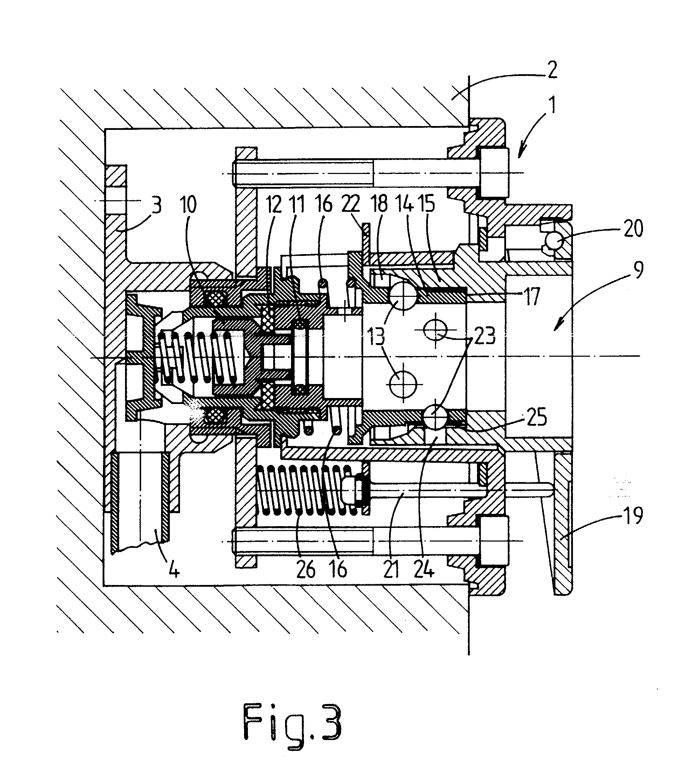

The drawing shows a device for connecting a user to a source of medical gas, such as for example oxygen or laughing-gas. The device comprises a connecting box 1 which is mounted within a wall 2 and through a connecting piece 3 is connected to a gas line 4 leading to a gas source or vacuum source which is located elsewhere. Further, the device comprises a partially shown connecting element 5 which is connected to a user not shown through a line 6. The connecting element comprises a connecting nipple 7 with a collar 8 extending circumferentially and projecting radially. The connecting element 5 is inserted with its connecting nipple 7 into a connecting opening 9 clearly shown in Fig. 3 of the connecting box 1 and the connecting element 5 is located in a working position in fig. 1, in which a closing valve 10 is opened, so that gas can be provided to the user through the connecting element 5 and the line 6. An O-ring 11 sealingly engages the end of the connecting nipple 7.

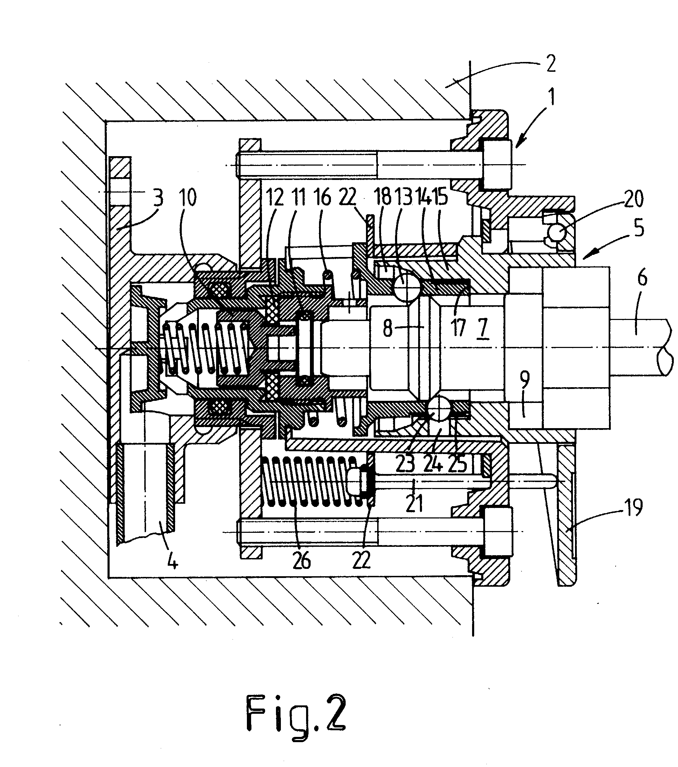

Fig. 2 shows the connecting element 5 located in a parking position within the connecting box, in which position the closing valve 10 sealingly engages a closing ring 12, so that the gas supply to the user is closed.

For locking the connecting element 5 in the working position within the connecting box 1 first locking elements 13 are provided, which in the embodiment described are made as balls and are distributed uniformally along the circumference of the connecting nipple 7. In Fig. 3 in which only the connecting box 1 is shown, two of the in this case three provided locking elements 13 are visible. These locking elements 13 are projecting into the connecting opening 9 and engage the collar 8 of the locking nipple 7 so that this nipple is not movable in axial direction. The locking elements 13 are mounted in a blocking ring 14 which is axially slidable in a mainly cylindrical coupling body 15. A spring 16 presses the blocking ring 14 against a shoulder 17 of the cylindrical coupling body 15. In this position of the blocking ring 14 the locking elements 13 are located in the blocking ring 14 blocked in radial direction, in that the locking elements 13 are abutting the wall of a recess 18 in the cylindrical body 15.

The blocking ring 14 can be moved axially against the action of the spring 16 by pressing a button 19 which is pivotably connected to the connecting box 1 at 20. The button 19 engages a pin 21 which is coupled with the blocking ring 14 through an annular plate 22. When the button 19 is pushed in, the blocking ring 14 moves inwardly in axial direction, whereby the locking elements 13 are allowed to move radially outwardly into the recess 18.

In this axially inwardly located position of the blocking ring 14, second locking elements 23 which are also mounted in this ring 14, are blocked against radial movement in that these locking elements are opposite of the inner wall of the cylindrical coupling body 15. In this manner it is obtained that the collar 8 is allowed to pass the locking elements 13, however, is not allowed to pass the locking elements 23 so that if the actuating button 19 is pushed in, the connecting element cannot be taken fully out of the connecting box 1. At a high gas pressure in the line 6, the connecting element 5 is therefore blocked by the locking elements 23 during pushing in the actuating button 19 and the connecting element cannot shoot out of the connecting box 1 unexpectedly.

In the rest position of the blocking ring 14 these locking elements 23 further provide a locking of the connecting element 5 in the parking position as shown in Fig. 2. In this parking position the locking elements 23 are located opposite of openings 24 in the coupling body 15. The locking elements 23 which are pressed radially inwardly by a spring ring 25, can move radially outwardly into the openings 24 by exerting a pulling force on the connecting element 5 so that the connecting element 5 can be removed from the connecting box 1 if desired. In the embodiment shown three locking elements 23 are used made as balls which are distributed uniformally along the circumference of the connecting nipples 7. Two balls 23 are shown in Figs. 2 and 3.

The actuating button 19 is maintained in the position shown in the drawings by a spring 26.

When the connecting element 5 is inserted into the connecting opening 9 of the connecting box 1, the collar 8 of the connecting nipple 7 can pass the locking elements 23, in that the blocking ring 14 is pressed fully inwardly and the locking elements 23 move radially outwardly into the recess 18 against the action of the spring ring 25. By pressing the connecting nipple 7 further into the connecting opening 9 the collar 8 presses the blocking ring 14 again inwardly through the locking elements 13, so that the locking elements 13 can move radially outwardly into the recess 18 of the cylindrical body 15 and the collar 8 is allowed to pass. The locking elements 13 will be located behind the collar 8 as soon as the spring 16 presses the blocking ring 14 against the shoulder 17 again.

The remaining part of the construction of the connecting box and the connecting element are not further described here as this is no part of the present invention.

From the foregoing it will be clear that the invention provides a device for connecting a user to a gas source, wherein it is guaranteed in an efficient manner that the connecting element 5 is secured against shooting unintentionally fully out of the connecting box 1 from its working position. Thereby unsafe situations can be avoided with certainty.

Although the locking elements 13 and 23 are made as balls in the above-described embodiment, they can also be made as rods or the like. Further, a different number instead of three locking elements 13, 23 can be provided.

## Claims

1. Device for connecting a user to a source of medical gas or a vacuum source, comprising a connecting box (1) to be connected to the gas source and having a closing valve (10), and a connecting element (5) to be connected with the user and having a connecting nipple (7) insertable in a connecting opening (9) of the connecting box and slidable within the connecting opening in axial direction between a parking position and a further inserted working position, wherein the closing valve is opened in the working position only, wherein the connecting nipple includes a collar (8) and the connecting opening includes first and second locking elements (13; 23) adapted to engage the collar of the connecting nipple to lock the connecting element in the connecting box in a non-working position and a working position, respectively, and wherein the connecting box is provided with a manually operable actuating means (19) for releasing the locking of the connecting element in the connecting box, **characterized** in that the actuating means (19) when releasing the locking of the connecting element (5) in the working position, activates locking means for blocking the connecting element (5) in the non-working position in that the second locking elements (23) are maintained in engagement with the collar (8) of the connecting nipple (7) in the non-working position by a spring (25) and during pulling the locking nipple outwardly are movable radially outwardly against the spring action, wherein the actuating means (19) when releasing the locking of the connecting element (5) in the working position, blocks the radial movement of the second locking elements (23).

2. Device according to claim 1, **characterized** in that the second locking elements (23) also function as said locking means.

3. Device according to claim 2, **characterized** in that the first and second locking elements (13; 23) are received in a blocking ring (14) located in a substantially cylindrical coupling body (15), wherein the blocking ring (14) and the cylindrical coupling body (15) are mutually slidable in axial direction by means of the actuating means (19), wherein the first locking elements (13) are movable in radial direction away from the collar (8) into a recess (18) in the cylindrical body (15) only at operating the actuating means (19) for releasing the locking.

4. Device according to claim 3, **characterized** in that blocking ring (14) is pressed into a working position by a spring (16), in which working position the first locking elements (13) are not radially movable, wherein by cooperation between the collar (8) of the connecting nipple (7) and the second and first locking elements (13; 23), respectively, the ring (14) is axially movable against the action of the spring (16) for radially moving the second and first locking elements (13; 23), respectively, into the recess (18) in the cylindrical body (15).

5. Device according to any one of the preceding claims, **characterized** in that the first and second locking elements (13; 23) each comprise a plurality of balls or the like which are distributed uniformally along the circumference of the connecting nipple (7).

## Patentansprüche

1. Vorrichtung zum Verbinden eines Benutzers mit einer Quelle medizinischen Gases oder einer Vakuum-Quelle, die ein Verbindungsbehältnis (1) zum Anschluß an eine Gasquelle umfaßt und ein Schließventil (10) besitzt sowie ein Verbindungselement (5), das mit dem Benutzer verbindbar ist, und das einen, in eine Verbindungsöffnung (9) des Verbindungsbehältnisses aufnehmbaren Verbindungsnippel (7) besitzt, der innerhalb der Verbindungsöffnung in achsialer Richtung zwischen einer Halteposition und einer weiter vorgerückten Arbeitsposition verschiebbar ist, wobei das Schließventil nur in der Arbeitsposition geöffnet ist, wobei der Verbindungsnippel einen Kragen (8) und die Verbindungsöffnung erste und zweite Arretierelemente (13, 23) umfaßt, die dazu geeignet sind, den Kragen des Verbindungsnippels zu beaufschlagen, um das Verbindungselement in dem Verbindungsbehältnis in einer Nicht-Arbeitsposition beziehungsweise einer Arbeitsposition zu arretieren, und wobei das Verbindungsbehältnis mit einem von Hand bedienbaren Betätigungsmittel (19) zum Lösen der Arretierung des Verbindungselementes in dem Verbindungsbehältnis ausgebildet ist, **dadurch gekennzeichnet**, daß das Betätigungsmittel (19) beim Lösen der Arretierung der Verbindungselemente (5) in der Arbeitsposition Arretiermittel zur Sperrung des Verbindungselementes (5) in der Nicht-Arbeitsposition aktiviert, insofern, als die zweiten Arretierelemente (23) in einer Nicht-Arbeitsposition durch eine Feder (25) mit dem Kragen (8) des Verbindungsnippels (7) in Verbindung gehalten werden, und sie, während man den Verriegelungsnippel nach außen zieht, entgegen der Wirkung der Feder radial nach außen hin bewegbar sind, wobei das Betätigungsmittel (19) die radiale Bewegung der zweiten Arretierelemente (23) beim Lösen der Arretierung des Verbindungselements (5) in der Arbeitsposition sperrt.

2. Vorrichtung nach Anspruch 1, **dadurch gekennzeichnet**, daß die zweiten Arretierelemente (23) ebenso wie die genannten Arretiermittel wirken.

3. Vorrichtung nach Anspruch 2, **dadurch gekennzeichnet**, daß die ersten und zweiten Arretierelemente (13; 23) in einem Sperring aufgenommen werden, der in einem im wesentlichen zylindrischen Kopplungskörper (15) angeordnet ist, wobei der Sperring (14) und der zylindrische Kopplungskörper (15) gegenseitig in achsialer Richtung durch das Betätigungsmittel (19) verschiebbar sind, wobei die ersten Arretierelemente (13) nur beim Ansprechen der Betätigungsmittel (19) zum Lösen der Arretierung in radialer Richtung vom Kragen (8) weg in eine Vertiefung im zylindrischen Körper (15) bewegbar sind.

4. Vorrichtung nach Anspruch 3, **dadurch gekennzeichnet**, daß der Sperring (14) durch eine Feder (16) in eine Arbeitsposition gedrückt wird, und in dieser Arbeitsposition die ersten Arrretierelemente (13) nicht radial bewegbar sind, wobei beim Zusammenwirken zwischen dem Kragen (8) des Verbindungsnippels (7) und den zweiten beziehungsweise ersten Arretierelementen (13; 23) der Ring (14) achsial entgegen der Wirkung der Feder (16) zur radialen Bewegung der zweiten beziehungsweise ersten Arretierelemente (13; 23) in die Vertiefung (18) des zylindrischen Körpers (15) bewegbar ist.

5. Vorrichtung nach einem der vorherigen Ansprüche, **dadurch gekennzeichnet**, daß die ersten und zweiten Arretierelemente (13; 23) jeweils eine Mehrzahl von Kugeln oder dergleichen aufweisen, die gleichmäßig entlang des Umfangs des Verbindungsnippels (7) verteilt sind.

## Revendications

1. Dispositif pour raccorder un utilisateur à une source de gaz médical ou à une source de vide, comprenant une boîte de raccordement (1) destinée à être raccordée à la source de gaz et ayant une vanne de fermeture (10), et un organe de raccordement (5) destiné à être raccordé à l'utilisateur et ayant un manchon de raccordement (7) insérable dans une ouverture de raccordement (9) de la boîte de raccordement et coulissant dans l'ouverture de raccordement dans une direction axiale entre une position de repos et une position active plus insérée, dans lequel la vanne de fermeture est ouverte uniquement dans la position active, dans lequel le manchon de raccordement comprend une bague (8) et l'ouverture de raccordement comprend des premiers et seconds organes de verrouillage (13 ; 23) adaptés pour coopérer avec la bague du manchon de raccordement pour verrouiller l'organe de raccordement dans la boîte de raccordement respectivement dans une position non-active et une position active, et dans lequel la boîte de raccordement est munie de moyens (19) d'actionnement utilisables manuellement pour libérer le verrouillage de l'organe de raccordement dans la boîte de raccordement, caractérisé en ce que, lors de la libération du verrouillage de l'organe de raccordement (5) dans la position active, les moyens (19) d'actionnement actionnent les moyens de verrouillage pour bloquer l'organe de raccordement (5) dans la position non-active, et en ce que les seconds organes de verrouillage (23) sont maintenus en coopération avec la bague (8) du manchon de raccordement (7)dans la position non-active par un ressort (25), et, pendant la poussée du manchon de verrouillage vers l'extérieur, sont déplaçables radialement vers l'extérieur contre l'action du ressort, dans lequel les moyens (19) d'actionnement, lors de la libération du verrouillage de l'organe de raccordement (5) dans la position active, bloquent le mouvement radial des seconds organes de verrouillage (23).

2. Dispositif selon la revendication 1, caractérisé en ce que les seconds organes de verrouillage (23) fonctionnent également comme lesdits moyens de verrouillage

3. Dispositif selon la revendication 2, caractérisé en ce que les premiers et seconds organes de verrouillage (13 ; 23) sont reçus dans un anneau de verrouillage (14) situé dans un corps d'accouplement (15) sensiblement cylindrique, dans lequel l'anneau de verrouillage (14) et le corps d'accouplement cylindrique (15) sont coulissants l'un par rapport à l'autre dans une direction axiale grâce aux moyens d'actionnement (19), dans lesquels les premiers organes de verrouillage (13) sont déplaçables dans une direction radiale en s'éloignant depuis la bague (8) dans une cavité (18) dans le corps cylindrique (15) seulement lors de l'utilisation des moyens d'actionnement (19) pour libérer le verrouillage.

4. Dispositif selon la revendication 3, caractérisé en ce que l'anneau de verrouillage (14) est pressé dans une position active par un ressort (16), les premiers organes de verrouillage (13) n'étant pas déplaçables radialement dans cette position active, dans lequel, par coopération entre la bague (8) du manchon de raccordement (7), et les premiers et seconds organes de verrouillage (13 ; 23) respectivement, l'anneau (14) est déplaçable axialement contre l'action du ressort (16) pour déplacer radialement les premiers et seconds organes de verrouillage (13 ; 23), respectivement, dans la cavité (18) dans le corps cylindrique (15).

5. Dispositif selon l'une quelconque des revendications précédentes, caractérisé en ce que les premiers et seconds organes (13 ; 23) de verrouillage comprennent chacun plusieurs billes ou analogue qui sont distribuées uniformément le long de la circonférence du manchon de raccordement (7).
